# EUROPEAN PATENT SPECIFICATION

(11) **EP 2 707 032 B1**
(45) Date of publication and mention of the grant of the patent: **26.06.2019**
(21) Application number: 12723023.3
(22) Date of filing: 10.05.2012
(51) Int. Cl.: A61K 47/34, A61K 9/00, A61K 31/4535, A61P 25/04

(54) **IMPLANTABLE POLYMERIC DEVICE FOR SUSTAINED RELEASE OF SUFENTANIL**
IMPLANTIERBARE POLYMERVORRICHTUNG ZUR VERZÖGERTEN FREISETZUNG VON SUFENTANIL
DISPOSITIF POLYMÈRE IMPLANTABLE POUR LA LIBÉRATION PROLONGÉE DE SUFENTANIL

(30) Priority: 10.05.2011 US 201161484222 P
(43) Date of publication of application: 19.03.2014
(73) Proprietor: Antecip Bioventures II LLC, Wilmington, DE 19808 (US)
(72) Inventor: TABUTEAU, Herriot, New York NY 10010 (US)
(74) Representative: Potter Clarkson
(86) International application number: PCT/US2012/037373
(87) International publication number: WO 2012/154986

(56) References cited:
- WO-A2-00/54745
- US-A1- 2004 033 250

## Description

### CROSS-REFERENCE TO RELATED APPLICATIONS

This application claims the benefit of United States Provisional Patent Application No. 61/484,222, entitled "IMPLANTABLE POLYMERIC DEVICE FOR SUSTAINED RELEASE OF SUFENTANIL," filed on May 10, 2011.

### BACKGROUND OF THE INVENTION

Sufentanil is a very potent opioid. Some have reported sufentanil to be about 5 to10 times as potent as fentanyl or 500 to 1,000 times as potent as morphine. It also has a much higher therapeutic index than fentanyl and morphine. These qualities, greater potency and potentially better safety, make it an attractive compound for clinical use. However its role has been largely restricted to the hospital setting since it is not orally bioavailable and so must be given parenterally (e.g. intravenously and intrathecally). In addition, since it has a relatively short half-life, it is not suitable for long term use.

Many patients with chronic pain require long-term continuous dosing with opioid analgesics. Effective treatment often necessitates the ingestion of multiple tablets per day. This situation can lead to poor compliance, inadequate pain relief and the development of addiction or dependence due to the peak and troughs in drug serum levels, and the potential for drug diversion for illicit use. In addition, patients on long-term oral therapy are subject other opioid-related side effects.

Addiction to heroin and other opioids has traditionally been treated with methadone, and more recently buprenorphine. Opioids with long plasma half-lives and that are orally or sublingually available (e.g. methadone and buprenorphine) have generally been used to treat opioid addiction. Because opioids such as methadone and buprenorphine are given orally or sublingually, they can be diverted for illicit use. In addition, compliance can be less than ideal. Sufentanil has not been used for the treatment of opioid dependence as it does not have a long plasma half-life and is not usually orally administered.

Sufentanil's potency and potential safety make it attractive for the treatment of chronic pain and opioid addiction and dependence if it could be delivered continuously outside of the hospital in a convenient way without the need for intravenous administration and its attendant costs and inconveniences. Thus, there is a need for a sufentanil formulation that will allow the drug to be used outside of the hospital setting for long-term continuous administration, and that will improve compliance, improve safety and reduce the potential for abuse and diversion.

WO 00/54745 describes the invention features devices and methods for the systemic delivery of fentanyl or a fentanyl congener (e.g., sufentanil) to treat pain. In the present invention, a drug formulation comprising fentanyl or a fentanyl congener is stored within a drug delivery device (e.g., contained in a reservoir or impregnated within a matrix within the controlled drug delivery device). The drug formulation comprises an amount of drug sufficient for treatment and is stable at body temperatures (i.e., no unacceptable degradation) for the entire pre-selected treatment period. The drug delivery devices store the drug formulation safely (e.g., without dose dumping), provide sufficient protection from bodily processes to prevent unacceptable degradation of the formulation, and release the drug formulation in a controlled fashion at a therapeutically effective rate to treat pain. In use, the drug delivery device is implanted in the subject's body at an implantation site, and the drug formulation is released from the drug delivery device to a delivery site. The delivery site may be the same as, near, or distant from the implantation site. Once released at the delivery site, the drug formulation enters the systemic circulation and is transported to the site of action in the body to modulate the pain response (e.g., the brain or other pain sensory location).

### SUMMARY

The present invention provides an implantable decide as described in claim 1. Devices comprising an opioid such as sufentanil and a polymeric matrix may allow sufentanil to be used for the treatment of pain or opioid addiction by maintaining a therapeutic level of sufentanil through extended release of the compound. The devices may also improve compliance and reduce the potential for diversion.

Some embodiments include an implantable device comprising: a solid biocompatible polymer matrix; and sufentanil encapsulated within the polymer matrix. The polymer matrix may comprise a plurality of pores configured to allow contact between the sufentanil and a physiological fluid of a mammal into which the device is implanted to thereby release sufentanil in vivo from the device into the mammal.

Also described is a method of treating pain or opioid addiction for an extended duration after a single administration of sufentanil, comprising: implanting one or more devices described herein into the body of a human being; wherein sufentanil is released from the devices into the body of the human being in
an amount of about 250 µg to about 300 µg of sufentanil per day for at least about 6 months.

Also described is a method of providing a constant serum concentration of sufentanil for an extended duration comprising: implanting one or more devices described herein into the body of a human being; wherein sufentanil is released from the devices into the body of the human being; and wherein the method provides a serum concentration of sufentanil in the human being in the range of about 10 pg/mL to about 50 pg/mL for at least about 6 months.

The implanting of one or more devices described herein into a mammal or human body to treat pain is also described herein.

The implanting one or more devices described herein into a mammal or human body to treat opioid addiction or dependency is also described herein.

### DETAILED DESCRIPTION

The implantable devices described herein may be implanted into any mammal, including a human. When the device is implanted, it may continuously release sufentanil in vivo over an extended duration. This may improve compliance with drug dosing regimens and reduce abuse potential because a physician implants the device. These devices may also provide more constant blood levels of sufentanil than injectable, sublingual or transdermal dosage forms, and may thus minimize side effects.

Because of sufentanil's high potency, a therapeutic effect may be provided over an extended period of time using only very small quantities of sufentanil. This may allow smaller and/or fewer devices to be used while still providing extended release of sufentanil. This may provide greater flexibility for incorporating sufentanil into a polymeric device suitable for implantation.

Generally, an implantable device may comprise sufentanil and a solid biocompatible polymer matrix. The implantable devices described herein do not require external medical equipment such as an intravenous (IV) system, to release the drug. Nor do these implantable devices require an internal or external mechanical pump.

The shape of the devices may be modified according to where the device is implanted and other factors. In some embodiments, devices may be disk shaped, square or rectangular chip-shaped, cylindrical, square or rectangular rod-shaped (e.g. the cross-section of the rod is square or rectangular), etc. Shapes may be altered by varying the shape of the die in the extruder, cutting the extruded material, or by injecting extruded or mixed material into a mold. The above descriptions of shapes may be approximations, and the actual devices may deviate significantly from the ideal shape as long as the shape is recognizable.

Devices may be sized to provide the desired amount of drug according to the loading of the drug. In some embodiments, the device has a diameter of about 0.5 mm to about 5 mm, about 2 mm to about 10 mm, about 2 mm to about 5, mm, about 1 mm to about 2 mm, about 2 mm to about 3 mm, about 3 mm to about 4 mm, or about 4 mm to about 5 mm. In some embodiments, the device may be rigid because the polymer is rigid and the diameter is too large for the device to be flexible.

In some embodiments, the device has a length of about 1 cm to about 10 cm, about 1 cm to about 5 cm, about 1 cm to about 2 cm, about 2 cm to about 3 cm, about 3 cm to about 4 cm, or about 4 cm to about 5 cm. In some embodiments, the device may have a mass of about 1 mg to about 10 g, about 10 mg to about 5 g, or about 25 mg to about 1000 mg, about 20 mg to about 50 mg, about 50 mg to about 100 mg, about 100 mg to about 150 mg, about 150 mg to about 200 mg, or about 50 mg to about 200 mg. In some embodiments, the device may have volume of about 0.01 mL to about 2 mL, about 0.05 mL to about 1 mL, about 0.05 mL to about 0.1 mL, about 0.1 mL to about 0.15 mL, about 0.15 mL to about 0.2 mL, about 0.2 mL to about 0.3 mL, or about 0.05 mL to about 0.3 mL.

Multiple implantable devices may be used. The size of the device and the number of devices implanted may depend upon the rate and duration of the sustained release desired. In some embodiments, the number of devices implanted into a human being may be 1 , 2, 3, 4, 5, 6, 7, 8, 9, 10, 1 1 , 12, 13, 14, or 15. In some embodiments, the total weight of all devices implanted is about 60 mg to about 1 g.

For delivery of sufentanil to a human being for about 6 months, 1 or 2 devices may be implanted, wherein the total weight of the devices may be about 20 mg to about 150 mg. For delivery of sufentanil to a human being for about 12 months, 1 , 2, 3, or 4 devices may be implanted, wherein the total weight of the devices may be about 40 mg to about 300 mg. For delivery of sufentanil to a human being for about 18 months, 1 , 2, 3, 4, 5, or 6 devices may be implanted, wherein the total weight of the devices may be about 60 mg to about 450 mg. For delivery of sufentanil to a human being for about 24 months, 1 , 2, 3, 4, 5, 6, 7, or 8 devices may be implanted, wherein the total weight of the devices may be about 80 mg to about 600 mg.

In some embodiments the number of devices implanted is 1 , 2, 3, 4, 5, 6, 7, 8, 9, 10, 1 1 , or 12 wherein each device has a weight of about 50 mg to about 100 mg. In some embodiments, the number of devices implanted is 2, 3, or 4.

While there are many ways that an implantable device may be fabricated, in some embodiments the device is prepared by a method comprising extruding a mixture of the polymer matrix and the sufentanil. The polymer and/or the sufentanil may be ground, mixed, and/or melted before extrusion. After extrusion, the material may be cut or injected into a mold. Methods that may be used include those described in US 7,736,665.

Implantable devices described herein may be used to treat opioid addiction and pain, including any pain condition which may require administration of an analgesic substance, e.g., postoperative pain, cancer pain, arthritic pain, lumbosacral pain, musculoskeletal pain, neuropathic pain, etc. This list, however, should not be interpreted as exhaustive. Generally, when a device is implanted subcutaneously, it may provide systemic drug delivery, so that it may relieve all types of pain the recipient may be suffering, and not just pain localized to a particular area, organ, or tissue of the body. To treat opioid addiction or pain includes diagnosis, cure, mitigation, treatment, or prevention of opioid addiction or pain in a mammal or human being.

Implantable devices are administered by implantation in an individual, generally by a physician or another health care provider. The device may be implanted subcutaneously in any of a variety of sites of the body, such as the upper arm, the back, the abdomen, etc. Multiple implantable devices may be administered, and the number of devices is one way that the dosage of sufentanil may be controlled. Furthermore, the length of time during which sufentanil is administered may be extended by re-implanting additional implantable devices in an individual receiving treatment before or after serum levels of sufentanil begin to decline, to maintain sufentanil at the desired level.

The polymer matrix of the devices may comprise a plurality of pores for the release of the drug. The pores may be formed by incorporation of sufentanil into the polymeric matrix. When a device is implanted, the pores in the device may allow contact between the sufentanil and a physiological fluid of a mammal or a human being. A physiological fluid includes any fluid that is naturally in the body of the mammal or human being, such as blood or interstitial fluid. When sufentanil comes in contact with the physiological fluid through the pores, part of the sufentanil may dissolve or disperse in the physiological fluid to provide in vivo release of sufentanil from the device into the systemic circulation. This may allow the sufentanil to come in contact with areas of the body where pain relief is needed. In some embodiments, the sufentanil may be circulated in the bloodstream.

An implantable device may provide a near constant amount of sufentantil, similar to an IV drip, but without the need for additional any equipment or personnel. Some devices have an initial burst release of sufentanil immediately following implantation, and after the burst may release a substantially constant amount of sufentanil. The burst period may be avoided or reduced by prewashing the device in a solvent or solution in which the sufentanil is soluble such as water, saline, or an organic solvent such as ethanol. The desired dosage rate may depend upon factors such as the underlying condition for which sufentanil is being administered, and the physiology of a particular patient.

The release rate of sufentanil can be altered by modifying parameters such as the percent drug loading, porosity of the matrix, structure of the implantable device, the hydrophobicity of the matrix, or the number of devices. A hydrophobic coating or a biodegradable coating may be placed over at least a portion of the device to further regulate the rate of release.

When implanted, the device or devices may release a therapeutically effective amount of sufentanil for an extended period of time after a single administration of sufentanil. A single administration of sufentanil includes administration of one or more devices or one or more dosage forms at substantially the same time, including for example, a single visit to a physician.

An extended period of time includes any period of time significantly longer than the amount of time an oral dosage form may provide a therapeutically effective amount of sufentanil after administration. In some embodiments, the devices may provide a therapeutically effective amount of sufentanil for at least about 1 month, at least about 3 months, at least about 6 months, at least about 9 months, at least about 1 year, at least about 15 months, at least about 18 months, at least about 2 years, about 1 month to about 3 years, about 3 months to about 2 years, about 3 months to about 6 months, about 6 months to about 9 months, about 9 months to about 1 year, about 1 year to about 15 months, about 15 months to about 18 months, about 18 months to about 21 months, about 21 months to about 24 months, about 3 months to about 1 year, about 6 months to about 1 year, or about 1 year to about 2 years. The extended delivery provided by some devices may reduce the need for external medical equipment and personnel associated with intravenous methods.

The amount of sufentanil released in vivo from the device or devices may vary according to the circumstances. In some embodiments, the device or devices may release sufentanil in vivo at a rate of about 20 µg/day to about 1 mg/day, about 100 µg/day to about 500 µg/day, about 100 µg/day to about 200 µg/day, about 200 µg/day to about 300 µg/day, about 300 µg/day to about 400 µg/day, or about 400 µg/day to about 500 µg/day. In some embodiments, the ratio of the average to the standard deviation of the amount of sufentanil released each day may be less than about 1, about 0.5, about 0.3, about 0.2, or about 0.1 for a time period of at least 1 month, at least about 2 months, at least about 3 months, or about 1 months to about 6 months after the device or devices are implanted. Since some devices have the burst period, this time period of substantially constant release may begin some time later than implantation, such as about 1 month, about 2 months, or about 3 months after implantation.

In some embodiments, the device or devices may be configured to provide a human serum concentration of sufentanil of about 0.5 pg/mL to about 500 pg/mL, about 1 pg/mL to about 200 pg/mL, about 5 pg/mL to about 10 pg/mL, or about 10 pg/mL to about 30 pg/mL, about 30 pg/mL to about 50 pg/mL, about 50 pg/mL to about 80 pg/mL, or about 5 pg/mL to about 80 pg/mL. Some devices may provide a substantially constant serum concentration of sufentanil after a burst period. In some embodiments, the ratio of the average to the standard deviation of the serum concentration taken daily may be less than about 1, about 0.5, about 0.3, about 0.2, or about 0.1 for a time period of at least 1 month, at least about 2 months, at least about 3 months, or about 1 month to about 6 months after the device is implanted. Since some devices have the burst period, this time period of substantially constant serum concentration may begin some time later than implantation, such as about 1 month, about 2 months, or about 3 months after implantation.

Sufentanil has the structure shown below.

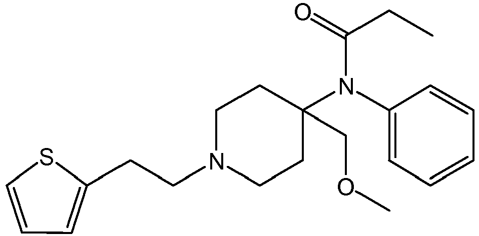

In an implantable device, sufentanil may be present as a free base or in any pharmaceutically acceptable salt form. In some embodiments, the sufentanil in the device is present as a free base, or as a salt, such as a citrate, lactate, tartrate, maleate, succinate, glycolate, phosphate, sulfate, or hydrochloride salt.

Some devices may use other opioids as a substitute for, or in conjunction with, sufentanil. Examples may include, but are not limited to, alfentanil, allyiprodine, alphaprodine, anileridine, benzylmorphine, bezitramide, buprenorphine, butorphanol, clonitazene, codeine, desomorphine, dextromoramide, dezocine, diampromide, diamorphone, dihydrocodeine, dihydromorphine, dimenoxadol, dimepheptanol, dimethylthiambutene, dioxaphetyl butyrate, dipipanone, eptazocine, ethoheptazine, ethyhnethylthiambutene, ethylmorphine, etonitazene, fentanyl, heroin, hydrocodone, hydromorphone, hydroxypethidine, isomethadone, ketobemidone, levorphanol, levophenacylmorphan, lofentanil, meperidine, meptazinol, metazocine, methadone, metopon, morphine, myrophine, narceine, nicomorphine, norlevorphanol, normethadone, nalorphine, nalbuphene, normorphine, norpipanone, opium, oxycodone, oxymorphone, papveretum, pentazocine, phenadoxone, phenomorphan, phenazocine, phenoperidine, piminodine, piritramide, propheptazine, promedol, properidine, propoxyphene, tilidine, tramadol and their pharmaceutically acceptable salts.

The amount of sufentanil in a device may vary depending upon circumstances such as the amount of sufentantil needed for the treatment, the duration that the sufentanil will need to be delivered, and the number of devices to be administered. In some embodiments, the device or devices implanted in a human being comprise about 1 mg to about 1000 mg, about 10 mg to about 500 mg, about 20 mg to about 50 mg, about 50 mg to about 80 mg, about 80 mg to about 120 mg, about 120 mg to about 150 mg, about 150 mg to about 200 mg, or about 20 mg to about 200 mg of sufentanil, such as sufentanil citrate or sufentanil free base.

The proportion of the sufentanil in the device may vary. Lower drug loading of the device or devices may allow slower release of the drug. Conversely, greater loading of the device or devices may allow more rapid release of the drug. This may be because a greater loading of the drug creates a larger number of pores in a device, thus increasing the release rate of the drug. In some embodiments, the sufentanil is about 10% to about 20%, about 30% to about 40%, about 40% to about 50%, about 60% to about 70%, about 70% to about 80%, about 80% to about 90%, or about 50% to about 75% of a device based upon weight.

In some embodiments, the rate of drug delivery in a device may be increased by including a water soluble filler to increase the number of pores in the extruded device. Any water soluble compound or salt that can be tolerated in a human or mammal body may be used. Examples of useful fillers may include, but are not limited to, sugars, such as glucose, mannose, galactose, lactose, fructose, sucrose, etc.; amino acids, such as glycine, alanine, valine, leucine, isoleucine, methionine, proline, phenylalanine, tryptophan, asparagine, glutamine, serine, threonine, aspartic acid, glutamic acid, tyrosine, cysteine, lysine, arginine, histidine, etc.; salts such as sodium chloride, phosphate salts, sulfate salts, etc.; organic acids such as citric acid, ascorbic acid, glycolic acid, etc; etc.

A biocompatible polymer matrix may be any polymeric material suitable for use in an mammal, including a human being. A biodegradable polymer matrix may disintegrate during delivery of the sufentanil so that it may not need to be removed after use. Examples of biodegradable polymer may include, but are not limited to: polyesters, polyorthoesters, polyphosphoesters, polycarbonates, polyanhydrides, polyphosphazenes, polyoxalates, polyaminoacids, polyhydroxyalkanoates, polyethyleneglycol, polyvinylacetate, polyhydroxyacids, polyanhydrides, copolymers and blends thereof, and the like. In some embodiments, a biodegradable polymer may be a co-polymer of lactic and glycolic acid.

A nondegradable polymer may also be used, including any biocompatible polymer that is substantially intact after release of sufentanil by a device is substantially complete. A nondegradable polymer may provide a more constant release of sufentanil than a biodegradable polymer. Examples of nondegradable polymers may include, but are not limited to: ethylene vinyl acetate copolymer (EVA), silicone, hydrogels such as crosslinked poly(vinyl alcohol) and poly(hydroxy ethylmethacrylate), acyl substituted cellulose acetates and alkyl derivatives thereof, partially and completely hydrolyzed alkylene-vinyl acetate copolymers, polyvinyl chloride, homo- and copolymers of polyvinyl acetate, polyethylene, polypropylene, crosslinked polyesters of acrylic acid and/or methacrylic acid, alkyl acrylates such as methyl methacrylate or methyl acrylate, polyacrylic acid, polyalkacrylic acids such as polymethacrylic acid, polyvinyl alkyl ethers, polyvinyl fluoride, polytetrafluoroethylene, polycarbonate, polyurethane, polyamide, polysulphones, polystyrene, styrene acrylonitrile copolymers, poly(ethylene oxide), poly(alkylenes), poly(vinyl imidazole), poly(esters), poly(ethylene terephthalate), polyphosphazenes, and chlorosulphonated polyolefins, and combinations thereof.

In some embodiments, the polymer matrix comprises an ethylene vinyl acetate copolymer. In some embodiments, the ethylene vinyl acetate copolymer comprises about 20% to about 50%, about 30% to about 40%, or about 30% to about 35% vinyl acetate by weight.

Any of the above polymers may also be used to coat a device in order to decrease the rate of release of the drug.

Some embodiments include a kit for use in opioid addiction or pain. The kits comprise at least one device described herein and instructions providing information regarding implantation of the device for treatment of opioid addiction or pain.

### Example 1

A mixture of 50% sufentanil citrate and 50% ethylene vinyl acetate (having 33% vinyl acetate) by weight is extruded through a die having a diameter around 2 mm. The temperature of the extruder and the die are about 200 °F to about 250 °F. The extruded mixture is cut into rods having a length of about 25 mm.

### Example 2

The procedure of Example 1 is repeated, except that the mixture is 60% sufentanil citrate and 40% ethylene vinyl acetate.

### Example 3

The procedure of Example 1 is repeated, except that the mixture is 70% sufentanil citrate and 30% ethylene vinyl acetate.

### Example 4

One extruded rod of Example 1 is implanted into an upper arm of a human patient to provide extended delivery of sufentanil to the patient.

### Example 5

One extruded rod of Example 2 is implanted into an upper arm of a human patient to provide extended delivery of sufentanil to the patient.

### Example 6

One extruded rod of Example 3 is implanted into an upper arm of a human patient to provide extended delivery of sufentanil to the patient.

### Example 7

Two extruded rods of Example 1 are implanted into an upper arm of a human patient to provide extended delivery of sufentanil to the patient.

### Example 8

Two extruded rods of Example 2 are implanted into an upper arm of a human patient to provide extended delivery of sufentanil to the patient.

### Example 9

Two extruded rods of Example 3 are implanted into an upper arm of a human patient to provide extended delivery of sufentanil to the patient.

### Example 10

Three extruded rods of Example 1 are implanted into an upper arm of a human patient to provide extended delivery of sufentanil to the patient.

### Example 11

Three extruded rods of Example 2 are implanted into an upper arm of a human patient to provide extended delivery of sufentanil to the patient.

### Example 12

Three extruded rods of Example 3 are implanted into an upper arm of a human patient to provide extended delivery of sufentanil to the patient.

### Example 13

Four extruded rods of Example 1 are implanted into an upper arm of a human patient to provide extended delivery of sufentanil to the patient.

### Example 14

Four extruded rods of Example 2 are implanted into an upper arm of a human patient to provide extended delivery of sufentanil to the patient.

### Example 15

Four extruded rods of Example 3 are implanted into an upper arm of a human patient to provide extended delivery of sufentanil to the patient.

## Claims

1. An implantable device comprising:
a solid nondegradable biocompatible polymer matrix;
a water soluble filler
sufentanil encapsulated within the polymer matrix;
wherein the polymer matrix comprises a plurality of pores configured to allow contact between the sufentanil and a physiological fluid of a mammal into which the device is implanted to thereby release sufentanil in vivo from the device into the mammal; and
wherein the implantable device is configured to achieve contact between physiological fluid and sufentanil inside the polymer matrix by entry of the physiological fluid through the pores.

2. The device of claim 1, wherein the polymer matrix comprises an ethylene vinyl acetate copolymer.

3. The device of claims 1 or 2, wherein the ethylene vinyl acetate copolymer comprises 20% to 50% vinyl acetate by weight, preferably 30% to 40% vinyl acetate by weight, more preferably 30% to 35% vinyl acetate by weight.

4. The device of claim 1, wherein the device comprises 20 mg to 200 mg of sufentanil citrate.

5. The device of claim 1, wherein the device comprises 20 mg to 200 mg of sufentanil free base.

6. The device of claim 1, wherein the device is configured to release sufentanil in vivo at a rate of 200 µg to 300 µg of the sufentanil per day.

7. The device of claim 1, wherein the device is configured to provide a human serum concentration of sufentanil of 5 pg/mL to 80 pg/mL.

8. The device of claim 1, wherein the sufentanil is 50% to 75% of the device based upon weight, preferably 60% to 70% of the device based upon weight.

9. The device of claim 1, wherein the device has a mass of 25 mg to 1000 mg.

10. The device of claim 1, wherein the device has a diameter of 2 mm to 5 mm.

11. The device of claim 1, wherein the device has a length of 1 cm to 5 cm.

12. The device of claim 1, wherein the device is configured to provide a therapeutically effective amount of sufentanil for a period of 3 months to 2 years.

13. The device of claim 1, wherein the water soluble filler is a sugar, an amino acid, a salt, or an organic acid;
preferably wherein
the sugar is glucose, mannose, galactose, lactose, fructose, or sucrose;
the amino acid is glycine, alanine, valine, leucine, isoleucine, methionine, proline, phenylalanine, tryptophan, asparagine, glutamine, serine, threonine, aspartic acid, glutamic acid, tyrosine, cysteine, lysine, arginine or histidine;
the organic acid is citric acid, ascorbic acid or glycolic acid;
the salt is sodium chloride, a phosphate or a sulfate; and
the solid nondegradable polymer matrix is ethylene vinyl acetate copolymer (EVA), silicone, hydrogels such as crosslinked poly(vinyl alcohol) and poly(hydroxy ethylmethacrylate), acyl substituted cellulose acetates and alkyl derivatives thereof, partially and completely hydrolyzed alkylene-vinyl acetate copolymers, polyvinyl chloride, homo- and copolymers of polyvinyl acetate, polyethylene, polypropylene, crosslinked polyesters of acrylic acid and/or methacrylic acid, alkyl acrylates such as methyl methacrylate or methyl acrylate, polyacrylic acid, polyalkacrylic acids such as polymethacrylic acid, polyvinyl alkyl ethers, polyvinyl fluoride, polytetrafluoroethylene, polycarbonate, polyurethane, polyamide, polysulphones, polystyrene, styrene acrylonitrile copolymers, poly(ethylene oxide), poly(alkylenes), poly(vinyl imidazole), poly(esters), poly(ethylene terephthalate), polyphosphazenes, and chlorosulphonated polyolefins, and combinations thereof.

14. A method for preparing the device of claim 1, wherein the method comprises extruding a mixture of the polymer matrix and the sufentanil.

15. A device for use in the treatment of pain or opioid addiction wherein the device comprises sufentanil and wherein the treatment comprises, after a single administration of sufentanil, implanting one or more devices according to any one of claims 1 to 13 into the body of a human being;
wherein sufentanil is released from the devices into the body of a human being in an amount of 250µg to 300µg of sufentanil per day for at least 6 months; and preferably wherein the number of devices implanted is 1 to 12, and more preferably 2 to 4.

## Patentansprüche

1. Implantierbare Vorrichtung, Folgendes umfassend:
eine feste, nicht abbaubare, biokompatible Polymermatrix;
einen wasserlöslichen Füllstoff
Sufentanil, der in der Polymermatrix ummantelt ist;
wobei die Polymermatrix mehrere Poren umfasst, die konfiguriert sind, um den Kontakt zwischen dem Sufentanil und einer physiologischen Flüssigkeit eines Säugers, in den die Vorrichtung implantiert ist, zu ermöglichen, um dadurch in vivo Sufentanil aus der Vorrichtung in das Säugergewebe freizusetzen; und
wobei die implantierbare Vorrichtung konfiguriert ist, um den Kontakt zwischen physiologischer Flüssigkeit und Sufentanil innerhalb der Polymermatrix durch Eintritt der physiologischen Flüssigkeit durch die Poren zu erreichen.

2. Vorrichtung nach Anspruch 1, wobei die Polymermatrix ein Ethylen-Vinylacetat-Copolymer umfasst.

3. Vorrichtung nach Anspruch 1 oder 2, wobei das Ethylen-Vinylacetat-Copolymer 20 bis 50 Gew.-% Vinylacetat, vorzugsweise 30 bis 40 Gew.-% Vinylacetat, stärker bevorzugt 30 bis 35 Gew.-% Vinylacetat umfasst.

4. Vorrichtung nach Anspruch 1, wobei die Vorrichtung 20 mg bis 200 mg Sufentanilcitrat umfasst.

5. Vorrichtung nach Anspruch 1, wobei die Vorrichtung 20 mg bis 200 mg freier Sufentanil-Base umfasst.

6. Vorrichtung nach Anspruch 1, wobei die Vorrichtung konfiguriert ist, um Sufentanil in vivo mit einer Rate von 200 µg bis 300 µg des Sufentanils pro Tag freizusetzen.

7. Vorrichtung nach Anspruch 1, wobei die Vorrichtung konfiguriert ist, um eine menschliche Serumkonzentration von Sufentanil von 5 µg/ml bis 80 µg/ml bereitzustellen.

8. Vorrichtung nach Anspruch 1, wobei das Sufentanil 50 % bis 75 % der Vorrichtung, bezogen auf das Gewicht, beträgt, vorzugsweise 60 % bis 70 % der Vorrichtung, bezogen auf das Gewicht.

9. Vorrichtung nach Anspruch 1, wobei die Vorrichtung eine Masse von 25 mg bis 1000 mg aufweist.

10. Vorrichtung nach Anspruch 1, wobei die Vorrichtung einen Durchmesser von 2 mm bis 5 mm aufweist.

11. Vorrichtung nach Anspruch 1, wobei die Vorrichtung eine Länge von 1 cm bis 5 cm aufweist.

12. Vorrichtung nach Anspruch 1, wobei die Vorrichtung konfiguriert ist, um eine therapeutisch wirksame Menge an Sufentanil für einen Zeitraum von 3 Monaten bis 2 Jahren bereitzustellen.

13. Vorrichtung nach Anspruch 1, wobei der wasserlösliche Füllstoff ein Zucker, eine Aminosäure, ein Salz oder eine organische Säure ist;
vorzugsweise wobei der Zucker Glukose, Mannose, Galaktose, Laktose, Fruktose oder Saccharose ist;
die Aminosäure Glyzin, Alanin, Valin, Leucin, Isoleucin, Methionin, Prolin, Phenylalanin, Tryptophan, Asparagin, Glutamin, Serin, Threonin, Asparaginsäure, Glutaminsäure, Tyrosin, Cystein, Lysin, Argininin oder Histidin ist;
die organische Säure Zitronensäure, Ascorbinsäure oder Glykolsäure ist;
das Salz Natriumchlorid, ein Phosphat oder ein Sulfat ist; und
die feste, nicht abbaubare Polymermatrix Ethylen-Vinylacetat-Copolymer (EVA), Silikon, Hydrogele wie vernetzter Poly(vinylalkohol) und Poly(hydroxyethylmethacrylat), acylsubstituierte Celluloseacetate und Alkylderivate davon, teilweise und vollständig hydrolysierte Alkylen-Vinylacetat-Copolymere, Polyvinylchlorid, Homo- und Copolymere von Polyvinylacetat, Polyethylen, Polypropylen, vernetzte Polyester von Acrylsäure und/oder Methacrylsäure, Alkylacrylate wie Methylmethacrylat oder Methylacrylat, Polyacrylsäure, Polyalkrylsäuren wie Polymethacrylsäure, Polyvinylalkylether, Polyvinylfluorid, Polytetrafluorethylen, Polycarbonat, Polyurethan, Polyamid, Polysulfone, Polystyrol, Styrol-Acrylnitril-Copolymere, Poly(ethylenoxid), Poly(alkylene), Poly(vinylimidazol), Poly(ester), Poly(ethylenterephthalat), Polyphosphazene und chlorosulfonierte Polyolefine und Kombinationen davon ist.

14. Verfahren zum Vorbereiten der Vorrichtung nach Anspruch 1, wobei das Verfahren das Extrudieren einer Mischung aus der Polymermatrix und dem Sufentanil umfasst.

15. Vorrichtung zur Verwendung bei der Behandlung von Schmerzen oder Opioidabhängigkeit, wobei die Vorrichtung Sufentanil umfasst und wobei die Behandlung nach einer einzigen Verabreichung von Sufentanil das Implantieren einer oder mehrerer Vorrichtungen nach einem der Ansprüche 1 bis 13 in den Körper eines Menschen umfasst;
wobei Sufentanil aus den Vorrichtungen in den Körper eines Menschen in einer Menge von 250 µg bis 300 µg Sufentanil pro Tag für mindestens 6 Monate freigesetzt wird; und vorzugsweise wobei die Anzahl der implantierten Vorrichtungen 1 bis 12 und stärker bevorzugt 2 bis 4 beträgt.

## Revendications

1. Dispositif implantable comprenant :
une matrice polymère biocompatible solide non dégradable ;
une charge de sufentanil hydrosoluble encapsulée dans la matrice polymère ;
la matrice polymère comprenant une pluralité de pores conçus pour permettre un contact entre le sufentanil et un fluide physiologique d'un mammifère dans l'organisme duquel le dispositif est implanté afin de libérer le sufentanil in vivo, du dispositif dans l'organisme du mammifère ; et
le dispositif implantable étant configuré pour établir un contact entre le fluide physiologique et le sufentanil à l'intérieur de la matrice polymère par l'entrée du fluide physiologique à travers les pores.

2. Dispositif selon la revendication 1, dans lequel la matrice polymère comprend un copolymère éthylène-acétate de vinyle.

3. Dispositif selon la revendication 1 ou 2, dans lequel le copolymère éthylène-acétate de vinyle comprend de 20 à 50 % en poids d'acétate de vinyle, de préférence de 30 à 40 % en poids d'acétate de vinyle et plus préférablement de 30 à 35 % en poids d'acétate de vinyle.

4. Dispositif selon la revendication 1, dans lequel le dispositif comprend de 20 à 200 mg de citrate de sufentanil.

5. Dispositif selon la revendication 1, dans lequel le dispositif comprend de 20 à 200 mg de base libre de sufentanil.

6. Dispositif selon la revendication 1, dans lequel le dispositif est configuré pour libérer le sufentanil in vivo à un taux de 200 à 300 µg de sufentanil par jour.

7. Dispositif selon la revendication 1, dans lequel le dispositif est configuré pour fournir une concentration sérique humaine de sufentanil en une proportion de 5 pg/mL à 80 pg/mL.

8. Dispositif selon la revendication 1, dans lequel le sufentanil représente 50 % à 75 % en poids du dispositif, de préférence 60 % à 70 % du poids du dispositif.

9. Dispositif selon la revendication 1, dans lequel le dispositif a une masse de 25 à 1000 mg.

10. Dispositif selon la revendication 1, dans lequel le dispositif a un diamètre de 2 à 5 mm.

11. Dispositif selon la revendication 1, dans lequel le dispositif a une longueur de 1 à 5 cm.

12. Dispositif selon la revendication 1, dans lequel le dispositif est configuré pour fournir une quantité thérapeutiquement efficace de sufentanil pendant une période de 3 mois à 2 ans.

13. Dispositif selon la revendication 1, dans lequel la charge hydrosoluble est un sucre, un acide aminé, un sel ou un acide organique ;
le sucre étant de préférence le glucose, le mannose, le galactose, le lactose, le fructose ou le saccharose ;
l'acide aminé étant la glycine, l'alanine, la valine, la leucine, l'isoleucine, la méthionine, la proline, la phénylalanine, le tryptophane, l'asparagine, la glutamine, la sérine, la thréonine, l'acide aspartique, l'acide glutamique, la tyrosine, la cystéine, la lysine, l'arginine ou l'histidine ;
l'acide organique étant l'acide citrique, l'acide ascorbique ou l'acide glycolique ;
le sel étant du chlorure de sodium, un phosphate ou un sulfate ; et
la matrice polymère solide non dégradable étant un copolymère éthylène-acétate de vinyle (EVA), du silicone, des hydrogels tels que l'alcool polyvinylique et le polyhydroxyéthylméthacrylate réticulés, des acétates de cellulose substitués par un acyle et leurs dérivés alkylés, des copolymères d'alkylène-acétate de vinyle partiellement et complètement hydrolysés, du chlorure de polyvinyle, homopolymères et copolymères d'acétate de polyvinyle, le polyéthylène, le polypropylène, des polyesters réticulés de l'acide acrylique et/ou de l'acide méthacrylique, des acrylates d'alkyle tels que le méthacrylate de méthyle ou l'acrylate de méthyle, un acide polyacrylique, des acides polyalkacryliques tels que l'acide polyméthacrylique, les polyvinylalkyléthers, le fluorure de polyvinyle, le polytétrafluoroéthylène, le polycarbonate, le polyuréthane, le polyamide, des polysulfones, le polystyrène, des copolymères de styrène-acrylonitrile, l'oxyde de polyéthylène, des polyalkylènes, le polyvinyl imidazole, des polyesters, le polyéthylène téréphtalate, des polyphosphazènes, des polyoléfines chlorosulphonées et leurs combinaisons.

14. Procédé de préparation du dispositif selon la revendication 1, le procédé comprenant l'extrusion d'un mélange de la matrice polymère et du sufentanil.

15. Dispositif destiné à être utilisé dans le traitement de la douleur ou de la dépendance aux opioïdes, le dispositif comprenant du sufentanil et le traitement comprenant, après une administration unique de sufentanil, l'implantation d'un ou de plusieurs dispositifs selon l'une des revendications 1 à 13 dans l'organisme d'un être humain ;
le sufentanil étant libéré des dispositifs dans l'organisme d'un être humain en une quantité comprise entre 250 et 300 µg de sufentanil par jour pendant au moins 6 mois, le nombre de dispositifs implantés étant de préférence compris entre 1 et 12, et plus préférablement entre 2 et 4.
